# FASCICULE DE BREVET EUROPEEN

(11) **EP 0 819 692 B1**
(45) Date de publication et mention de la délivrance du brevet: **16.08.2000**
(21) Numéro de dépôt: 97401459.9
(22) Date de dépôt: 23.06.1997
(51) Int. Cl.: C07D 417/12, A61K 7/48

(54) **Dérivé de l'acide kojique et son utilisation comme agent dépigmentant**
Kojic Säure Derivate als Hautpigmentierungsmittel
Kojic acid derivative as depigmentation agent

(30) Priorité: 18.07.1996 FR 9609011
(43) Date de publication de la demande: 21.01.1998
(73) Titulaire: L'OREAL, 75008 Paris (FR)
(72) Inventeur: Galey, Jean-Baptiste,, 93600 Aulnay-Sous-Bois (FR); Pichaud, Patrick, 78140 Velizy (FR)
(74) Mandataire: Lhoste, Catherine

(56) Documents cités:
- EP-A- 0 650 725
- EP-A- 0 780 120
- GB-A- 2 175 584
- US-A- 5 208 249
- CHEMICAL ABSTRACTS, vol. 121, no. 10, 5 septembre 1994 Columbus, Ohio, US; abstract no. 117364e, page 589; XP002027457 & JP 06 133 773 A (DAIICHI SEIYAKU CO) 17 mai 1994

## Description

La présente invention se rapporte à un nouveau dérivé de l'acide kojique et à son utilisation dans une composition à application topique, comme agent dépigmentant et/ou blanchissant de la peau humaine.

La couleur de la peau est fonction de différents facteurs et notamment des saisons de l'année, de la race et du sexe, et elle est principalement déterminée par la concentration de mélanine produite par les mélanocytes. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

Depuis plusieurs années, on cherche à diminuer et/ou ralentir la production de mélanine en vue de dépigmenter ou blanchir la peau en agissant sur un ou plusieurs des points de la synthèse biochimique intracellulaire de la mélanine.

Aussi depuis un bon nombre d'années, différentes molécules ont déjà été utilisées et testées comme agent dépigmentant ou blanchissant.

Le mécanisme de formation de la pigmentation de la peau, c'est-à-dire de la formation de la mélanine est particulièrement complexe et fait intervenir schématiquement les principales étapes suivantes :
Tyrosine ---> Dopa ---> Dopaquinone ---> Dopachrome ---> Mélanine

La tyrosinase est l'enzyme essentielle intervenant dans cette suite de réactions. Elle catalyse notamment la réaction de transformation de la tyrosine en Dopa (dihydroxyphénylalanine) et la réaction de transformation de la Dopa en dopaquinone. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut ainsi être bloquée et assurer la dépigmentation.

Les substances les plus utilisées en tant que dépigmentants dans des compositions sont notamment des composés tels que la vitamine C, les dérivés de vitamine C ou de vitamine E, l'arbutine, l'hydroquinone, l'acide kojique, les dérivés placentaires, le glutathion et ses dérivés.

Ces différents composés sont connus pour agir sur la synthèse et/ou l'activité de la tyrosinase, enzyme qui entre en jeu dans la synthèse de la mélanine, ou pour réduire la quantité de mélanine formée ou encore pour stimuler l'élimination de la mélanine au travers des kératinocytes. Malheureusement, ils sont soit toxiques, cas de l'hydroquinone, soit instables en solution, cas de la vitamine C et de l'acide kojique ce qui complique quelque peu la fabrication de la composition, soit encore peuvent présenter des odeurs désagréables et notamment des odeurs soufrées en ce qui concerne le glutathion, ce qui en limite par conséquent l'utilisation. De plus le nombre de ces composés est très limité.

Aussi, il subsiste le besoin d'un nouvel agent blanchissant de la peau, à action aussi efficace que ceux connus mais n'ayant pas leurs inconvénients, c'est-à-dire qui soit stable dans une composition, non toxique pour la peau et qui ne présente pas d'odeur désagréable et ce surtout dans la mesure où cet agent est appliqué sur la peau.

La demanderesse a trouvé de manière inattendue un composé dérivé de l'acide kojique, présentant la propriété d'inhiber la synthèse de la mélanine, et susceptible d'agir ainsi sur la pigmentation et les taches de la peau sans toxicité.

L'invention a donc pour objet le 2-oxo-thiazolidine-4-carboxylate de 5-hydroxy-4-oxo-4H-pyran-2-ylméthyle de formule (I) : 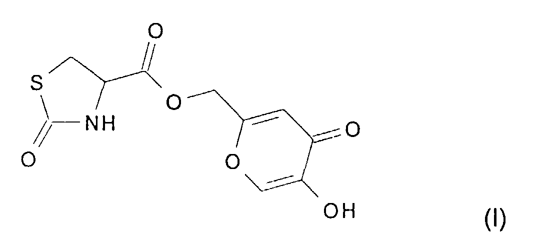 et ses sels pharmaceutiquement acceptables.

Ce composé est obtenu par réaction de l'acide kojique avec l'acide 2-oxothiazolidine-4-carboxylique. Ses sels peuvent être obtenus par réaction du composé de formule (I) avec des bases ou acides minéraux ou organiques, tels que l'acide chlorhydrique, l'acide acétique, la soude et la triéthanolamine.

La demanderesse a trouvé de façon surprenante que ce composé avait l'avantage de posséder une activité dépigmentante supérieure à celle de l'acide kojique tout en n'en présentant pas les inconvénients.

L'invention a aussi pour objet un procédé de fabrication du composé de formule (1), caractérisé en ce qu'il consiste à estérifier la fonction alcool de l'acide kojique avec l'acide L-2-oxothiazolidine 4-carboxylique.

De manière plus particulière, ce procédé consiste à préparer le dérivé bromé de l'acide kojique et à faire réagir ce dérivé bromé avec l'acide L-2-oxothiazolidine 4-carboxylique.

Ce procédé de fabrication consiste de manière plus détaillée à :
- mélanger l'acide kojique solubilisé en milieu acide, avec de l'acide bromhydrique, chauffer le mélange réactionnel, le refroidir et le filtrer ;
- faire réagir le dérivé bromé obtenu avec l'acide L-2-oxothiazolidine 4-carboxylique, en présence de carbonate de potassium ;
- chauffer le mélange réactionnel, le refroidir et le filtrer.

Le procédé de fabrication du composé (I) suit le schéma général suivant :

### Première étape :

Au cours de la première étape, on fait réagir l'acide kojique en solution dans l'acide sulfurique avec de l'acide bromhydrique selon l'équation suivante :

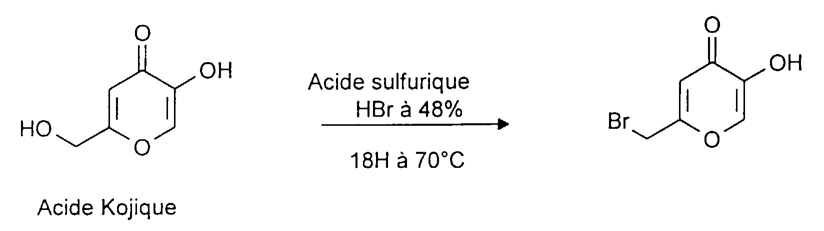

On solubilise 5 g d'acide kojique dans 15 ml d'acide sulfurique concentré. On contrôle l'exotherme par un bain d'eau et de glace. On ajoute lentement 20 ml d'acide bromhydrique à 48%. On observe un dégagement gazeux orange. On chauffe ensuite à 70°C pendant 18 heures.

On laisse la réaction refroidir vers 40°C, puis on coule le milieu dans 110 g d'un mélange d'eau et de glace. Un produit légèrement marron précipite. Après 1 heure sous agitation, on filtre sur verre fritté et on lave le solide obtenu par 10 ml d'eau. On sèche le produit au dessicateur sous vide à 50°C. On récupère 6,7 g d'un solide marron clair. Rdt_{brut} = 93%.

Le produit est utilisé tel quel pour la réaction suivante ou peut être purifié par réempatage à chaud dans l'eau (CCM : Rf = 0,7 dans le système éluant CH₂Cl₂ (9) / MeOH (1))

Le RMN ¹H du produit obtenu est conforme à la structure attendue.

### Deuxième étape :

Dans la seconde étape, on fait réagir le produit obtenu dans la première étape avec de l'acide L-2-oxothiazolidine 4-carboxylique :

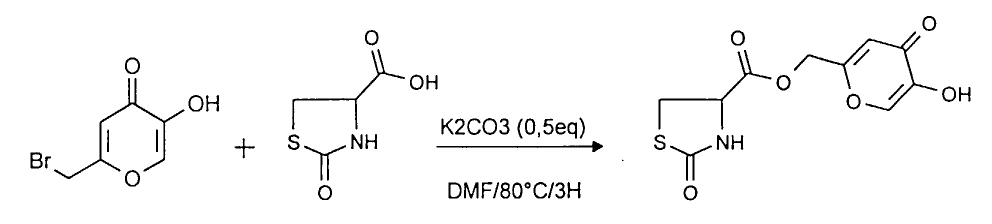

Dans un réacteur de 50 ml, on introduit 20,4 mmoles d'acide 2-oxothiazolidine 4-carboxylique dans 10 ml de DMF anhydre (solubilisation rapide). On ajoute 0,5 eq de K₂CO₃ et on chauffe à 80°C pendant 20 minutes. On additionne alors 21,4 mmoles du dérivé bromé isolé lors de la première étape. Les sels précipitent rapidement. Après 3 heure de réaction, on filtre pour éliminer l'insoluble et on évapore à sec. On obtient une gomme. On reprend cette dernière dans 100 ml d'eau et on laisse 1 heure sous agitation, puis on filtre sur verre fritté. Le solide obtenu est séché au dessicateur sous vide à 50°C. Masse obtenue: 2 g. Rdt_{brut} = 36%.

On effectue un réempatage à chaud du solide dans 25 ml de méthanol. On laisse 1 heure à reflux, puis on laisse ensuite revenir à la température ambiante. On filtre sur verre fritté ; ensuite, on sèche le solide au dessicateur sous vide à 50°C. Masse récupérée: 0,8 g. Rdt = 15%.

Le RMN ¹H du produit obtenu est conforme à la structure attendue.

L'analyse élémentaire du produit obtenu est la suivante :

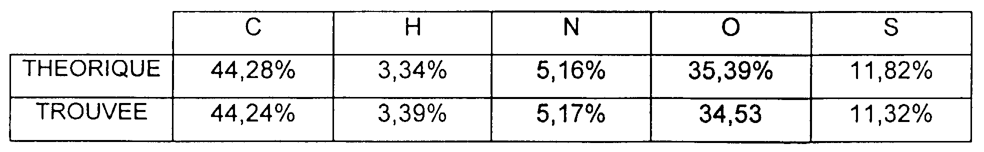

Comme indiqué ci-dessus, ce procédé de fabrication peut être éventuellement complété par une étape de préparation d'un sel.

Par ailleurs, un test a mis en évidence l'activité du composé de formule (I) comme inhibiteur de la tyrosinase par évaluation de son effet sur l'activité dopa-oxydase de la tyrosinase des mélanocytes humains.

Des mélanocytes humains ont été préparés et mis en culture, puis introduits dans des milieux comportant différentes concentrations d'ester (0,1 mM, 0,2 mM, 0,3 mM et 0,6 mM).

Après trois jours, les mélanocytes ont été « trypsinisés » dans un mélange de trypsine / EDTA 0,05 % / 0,02 % dans un tampon phosphate (50 mM à pH 6,8), lavés à trois reprises dans le tampon phosphate contenant 1 % (w/v) de Triton x-100 et soumis à des ultrasons. Après centrifugation, des échantillons ont été prélevés et mélangés avec de la L-DOPA 5 mM dans du tampon phosphate. L'activité en tyrosinase a été évaluée par spectrophotométrie en mesurant l'augmentation de l'absorbance à 475 nm due à la formation de dopachrome à partir de DOPA 5 mM. La lecture de l'absorbance a été effectuée sur un lecteur de microplaques à 25°C pendant 60 minutes.

L'activité de la tyrosinase cellulaire est mesurée par rapport à une gamme étalon réalisée avec la tyrosinase commerciale.

On détermine la valeur de IC₅₀, c'est-à-dire la concentration nécessaire en produit actif pour obtenir 50 % d'inhibition de la production de la mélanine. Pour le 2-oxothiazolidine-4-carboxylate de 5-hydroxy-4-oxo-4H-pyran-2-ylméthyle, cette valeur IC₅₀ est de 0,2 à 0,3 mM, alors qu'elle est de 1 à 2 mM pour l'acide kojique.

Par conséquent, le composé selon l'invention est plus actif que l'acide kojique.

L'invention a encore pour objet une composition contenant le composé de formule (I) tel que défini ci-dessus.

Selon une réalisation préférée de l'invention, cette composition contient un milieu physiologiquement acceptable, c'est-à-dire compatible avec la peau, le cuir chevelu et les cheveux, et constitue plus particulièrement une composition à application topique, cosmétique et/ou dermatologique, notamment blanchissante et/ou dépigmentante.

L'invention a aussi pour objet l'utilisation du composé de formule (I) tel que défini ci-dessus dans une composition cosmétique comme agent dépigmentant et/ou blanchissant de la peau humaine.

L'invention a aussi pour objet l'utilisation du composé de formule (I) tel que défini ci-dessus à titre de médicament.

L'invention a encore pour objet l'utilisation du composé de formule (I) tel que défini ci-dessus, pour la préparation d'une composition dermatologique destinée à la dépigmentation et/ou au blanchiment de la peau humaine.

L'invention a aussi pour objet un procédé de traitement cosmétique et/ou dermatologique, caractérisé en ce qu'il consiste à appliquer sur la peau le composé de formule (I) tel que défini ci-dessus, dans un milieu physiologiquement acceptable.

Enfin, l'invention a pour objet un procédé cosmétique et/ou dermatologique de dépigmentation et/ou blanchiment de la peau humaine, caractérisé en ce qu'il consiste à appliquer sur la peau le composé de formule (I) tel que défini ci-dessus, dans un milieu physiologiquement acceptable.

Le 2-oxo-thiazolidine-4-carboxylate de 5-hydroxy-4-oxo-4H-pyran-2-ylméthyle ou son sel peut être présent dans la composition selon l'invention en une quantité allant de 0,01 à 10 % en poids et de préférence de 0,1 à 5 % du poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage.

La composition selon l'invention peut comprendre tous les ingrédients classiquement utilisés dans le domaine cosmétique ou dermatologique, dans des concentrations habituelles. Ces ingrédients sont en particulier choisis parmi les corps gras, les conservateurs, les vitamines, les gélifiants, les parfums, les tensioactifs, l'eau, les antioxydants, les charges, les hydratants, les filtres et leurs mélanges.

Comme corps gras utilisables dans l'invention, on peut citer les huiles minérales (huile de vaseline), les huiles d'origine végétale (huile de jojoba), les huiles d'origine animale, les huiles de synthèse (palmitate d'isopropyle), les huiles siliconées (cyclopentadiméthylsiloxane) et les huiles fluorées. On peut aussi utiliser des alcools gras (alcool stéarylique), des acides gras, des cires.

Comme tensioactifs utilisables dans l'invention, on peut citer par exemple les esters d'acide gras et de polyéthylène glycol tels que le stéarate de polyéthylèneglycol, et les esters d'acide gras tels que le stéarate de sodium.

L'invention va maintenant être illustrée à l'aide des exemples qui suivent. Les concentrations sont données en pourcentage en poids.

### Exemple 1: crème blanchissante pour le visage

- 2-oxo-thiazolidine-4-carboxylate de 5-hydroxy-4-oxo-4H-pyran-2-ylméthyle 1 %
- stéarate de sodium 3 %
- huile de vaseline 6 %
- conservateur 1 %
- cyclopentadiméthylsiloxane 2 %
- alcool stéarylique 1 %
- parfum 1 %
- eau qsp 100 %

La crème obtenue utilisée en application quotidienne, permet d'obtenir un blanchiment de la peau.

### Exemple 2: crème blanchissante pour le corps

- 2-oxo-thiazolidine-4-carboxylate de 5-hydroxy-4-oxo-4H-pyran-2-ylméthyle 2.5 %
- huile de jojoba 13 %
- cire de sipol 6 %
- palmitate d'isopropyle 2 %
- stéarate de polyéthylène glycol 3 %
- glycérol (hydratant) 15 %
- conservateur 0.5 %
- parfum 1 %
- eau qsp 100 %

La crème obtenue peut être utilisée quotidiennement et est apte à dépigmenter la peau.

## Revendications

1. 2-oxo-thiazolidine-4-carboxylate de 5-hydroxy-4-oxo-4H-pyran-2-ylméthyle de formule (I) : 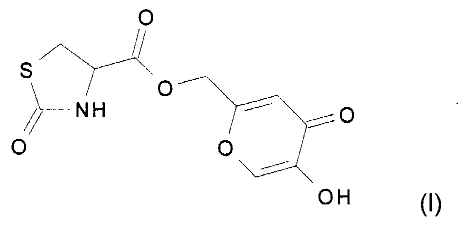 et ses sels pharmaceutiquement acceptables.

2. Composition, caractérisée en ce qu'elle comprend le composé selon la revendication 1.

3. Composition selon la revendication 2, caractérisée en ce qu'elle contient en outre un milieu physiologiquement acceptable.

4. Composition selon la revendication 2 ou 3, caractérisée en ce qu'elle consiste en une composition à application topique.

5. Composition selon l'une quelconque des revendications 2 à 4, caractérisée en ce que le composé de formule (I) est présent en une quantité allant de 0,01 à 10% du poids total de la composition.

6. Composition selon l'une quelconque des revendications 2 à 5, caractérisée en ce qu'elle comprend, en outre, au moins un ingrédient choisi parmi les corps gras, les conservateurs, les vitamines, les gélifiants, les parfums, les tensioactifs, l'eau, les antioxydants, les charges, les filtres, les hydratants et leurs mélanges.

7. Composition selon la revendication 6, caractérisée en ce que le corps gras est choisi parmi les huiles et les cires.

8. Composition selon la revendication 6 ou 7, caractérisée en ce que le tensioactif est choisi parmi le stéarate de sodium et le stéarate de polyéthylène glycol.

9. Composition selon l'une quelconque des revendications 2 à 8, caractérisée en ce qu'elle consiste en une composition dépigmentante ou blanchissante.

10. Composé selon la revendication 1 pour son utilisation comme médicament.

11. Utilisation du composé selon la revendication 1, pour la préparation d'une composition dermatologique destinée à la dépigmentation et/ou au blanchiment de la peau humaine.

12. Utilisation du composé selon la revendication 1 à titre d'agent cosmétique, dans un milieu physiologiquement acceptable.

13. Utilisation du composé selon la revendication 1 à titre d'agent cosmétique, dans un milieu physiologiquement acceptable, en vue de dépigmenter et/ou blanchir la peau humaine.

14. Procédé de fabrication du composé selon la revendication 1, caractérisé en ce qu'il consiste à estérifier la fonction alcool de l'acide kojique avec l'acide L-2-oxothiazolidine 4-carboxylique.

15. Procédé selon la revendication 14, caractérisé en ce que l'on prépare le dérivé bromé de l'acide kojique et on fait réagir ce dérivé bromé avec l'acide L-2-oxothiazolidine 4-carboxylique.

16. Procédé selon la revendication 14 ou 15, caractérisé en ce qu'il consiste à :
- mélanger l'acide kojique solubilisé en milieu acide, avec de l'acide bromhydrique, chauffer le mélange réactionnel, le refroidir et le filtrer ;
- faire réagir le dérivé bromé obtenu avec l'acide L-2-oxothiazolidine 4-carboxylique, en présence de carbonate de potassium ;
- chauffer le mélange réactionnel, le refroidir et le filtrer ;
- éventuellement préparer un sel du composé obtenu.

## Patentansprüche

1. 5-Hydroxy-4-oxo-4H-pyran-2ylmethyl-2-oxo-thiazolidin-4-carboxylat der Formel (I): 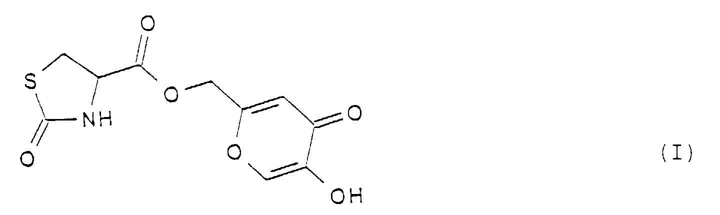 und seine pharmazeutisch akzeptablen Salze.

2. Zusammensetzung, dadurch gekennzeichnet, daß sie die Verbindung nach Anspruch 1 enthält.

3. Zusammensetzung nach Anspruch 2, dadurch gekennzeichnet, daß sie ferner ein physiologisch akzeptables Medium enthält.

4. Zusammensetzung nach Anspruch 2 oder 3, dadurch gekennzeichnet, daß es sich um eine Zusammensetzung zur topischen Anwendung handelt.

5. Zusammensetzung nach einem der Ansprüche 2 bis 4, dadurch gekennzeichnet, daß die Verbindung der Formel (I) in einem Mengenanteil von 0,01 bis 10 % des Gesamtgewichts der Zusammensetzung vorliegt.

6. Zusammensetzung nach einem der Ansprüche 2 bis 5, dadurch gekennzeichnet, daß sie ferner mindestens einen Bestandteil enthält, der unter den Fettsubstanzen, Konservierungsmitteln, Vitaminen, Gelbildnern, Parfums, grenzflächenaktiven Stoffen, Wasser, Antioxidantien, Füllstoffen, Filtern, Hydratisierungsmitteln und deren Gemischen ausgewählt ist.

7. Zusammensetzung nach Anspruch 6, dadurch gekennzeichnet, daß die Fettsubstanz unter den Ölen und Wachsen ausgewählt ist.

8. Zusammensetzung nach Anspruch 6 oder 7, dadurch gekennzeichnet, daß der grenzflächenaktive Stoff unter Natriumstearat und Polyethylenglykolstearat ausgewählt ist.

9. Zusammensetzung nach einem der Ansprüche 2 bis 8, dadurch gekennzeichnet, daß es sich um eine depigmentierende oder bleichende Zusammensetzung handelt.

10. Verbindung nach Anspruch 1 zur Verwendung als Arzneimittel.

11. Verwendung der Verbindung nach Anspruch 1 zur Herstellung einer dermatologischen Zusammensetzung, die die menschliche Haut depigmentieren oder bleichen soll.

12. Verwendung der Verbindung nach Anspruch 1 als kosmetisches Mittel in einem physiologisch akzeptablen Medium.

13. Verwendung der Verbindung nach Anspruch 1 als kosmetisches Mittel in einem physiologisch akzeptablen Medium, um die menschliche Haut zu depigmentieren und/oder zu bleichen.

14. Verfahren zur Herstellung einer Verbindung nach Anspruch 1, dadurch gekennzeichnet, daß es darin besteht, die Alkoholgruppe der Kojisäure mit der L-2-Oxothiazolidin-4-carbonsäure zu verestern.

15. Verfahren nach Anspruch 14, dadurch gekennzeichnet, daß das Bromderivat der Kojisäure hergestellt wird und dieses bromierte Derivat mit der L-2-Oxothiazolidin-4-carbonsäure umgesetzt wird.

16. Verfahren nach Anspruch 14 oder 15, dadurch gekennzeichnet, daß es darin besteht:
- die Kojisäure, die in einem sauren Medium solubilisiert ist, mit Bromwasserstoffsäure zu vermischen, und das Reaktionsgemisch zu erwärmen, abzukühlen und zu filtrieren;
- das erhaltene bromierte Derivat mit L-2-Oxothiazolidin-4-carbonsäure in Gegenwart von Kaliumcarbonat umzusetzen;
- das Reaktionsgemisch zu erwärmen, abzukühlen und zu filtrieren; und
- gegebenenfalls ein Salz der erhaltenen Verbindung herzustellen.

## Claims

1. 5-Hydroxy-4-oxo-4H-pyran-2-ylmethyl 2-oxothiazolidine-4-carboxylate of formula (I): 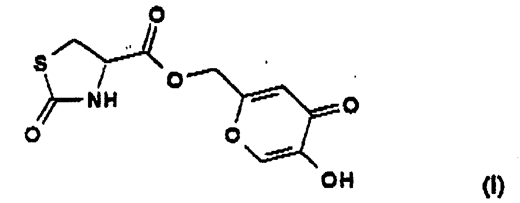 and the pharmaceutically acceptable salts thereof.

2. Composition, characterized in that it comprises the compound according to Claim 1.

3. Composition according to Claim 2, characterized in that it also contains a physiologically acceptable medium.

4. Composition according to Claim 2 or 3, characterized in that it consists of a composition for topical application.

5. Composition according to any one of Claims 2 to 4, characterized in that the compound of formula (I) is present in an amount ranging from 0.01 to 10 % of the total weight of the composition.

6. Composition according to any one of Claims 2 to 5, characterized in that it also comprises at least one ingredient chosen from fatty substances, preserving agents, vitamins, gelling agents, fragrances, surfactants, water, antioxidants, fillers, screening agents, wetting agents and mixtures thereof.

7. Composition according to Claim 6, characterized in that the fatty substance is chosen from oils and waxes.

8. Composition according to Claim 6 or 7, characterized in that the surfactant is chosen from sodium stearate and polyethylene glycol stearate.

9. Composition according to any one of Claims 2 to 8, characterized in that it consists of a depigmenting or bleaching composition.

10. Compound according to Claim 1 for its use as a medicinal product.

11. Use of the compound according to Claim 1 for the preparation of a dermatological composition intended for depigmenting and/or bleaching human skin.

12. Use of the compound according to Claim 1 as a cosmetic agent, in a physiologically acceptable medium.

13. Use of the compound according to Claim 1 as a cosmetic agent, in a physiologically acceptable medium, in order to depigment and/or bleach human skin.

14. Process for the manufacture of the compound according to Claim 1, characterized in that it consists in esterifying the alcohol function of kojic acid with L-2-oxothiazolidine-4-carboxylic acid.

15. Process according to Claim 14, characterized in that the bromo derivative of kojic acid is prepared and this bromo derivative is reacted with L-2-oxothiazolidine-4-carboxylic acid.

16. Process according to Claim 14 or 15, characterized in that it consists in:
- mixing kojic acid, dissolved in acidic medium, with hydrobromic acid, heating the reaction mixture, cooling it and filtering it;
- reacting the bromo derivative obtained with L-2-oxothiazolidine-4-carboxylic acid, in the presence of potassium carbonate;
- heating the reaction mixture, cooling it and filtering it;
- optionally preparing a salt of the compound obtained.
